(19) Europäisches Patentamt
European Patent Office
Office européen des brevets

(11) Publication number: 0 405 784 A1

(12) **EUROPEAN PATENT APPLICATION**

(21) Application number: 90306389.9

(51) Int. Cl.[5]: C07D 401/06, A61K 31/47

(22) Date of filing: 12.06.90

(30) Priority: 13.06.89 JP 148480/89

(43) Date of publication of application: 02.01.91 Bulletin 91/01

(84) Designated Contracting States: AT BE CH DE DK ES FR GB GR IT LI LU NL SE

(71) Applicant: ONO PHARMACEUTICAL CO., LTD.
1-5, Doshomachi 2-chome
Chuo-ku Osaka 541(JP)

(72) Inventor: Okegawa, Tadao, C/o Minase Research Institute
Ono Pharmaceutical Co. Ltd., 3-1-1 Sakurai
Shimamoto-cho, Mishima-gun, Osaka(JP)
Inventor: Kawamura, Masanori, C/o Minase Research Institute
Ono Pharmaceutical Co. Ltd., 3-1-1 Sakurai
Shimamoto-cho, Mishima-gun, Osaka(JP)

(74) Representative: Bentham, Stephen et al
J.A. Kemp & Co. 14 South Square Gray's Innlnn
London WC1R 5EU(GB)

(54) **Novel benzisoquinoline derivatives.**

(57) Benzisoquinoline derivatives of the formula:

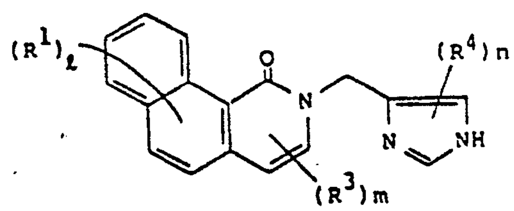

(I)

wherein $R^1$ is hydrogen, C1-4 alkyl, C1-4 alkoxy, hydroxy or halogen;
$R^3$ is hydrogen or C1-4 alkyl;
$R^4$ is hydrogen or C1-6 alkyl;
l is 1-6;
m is 1 or 2:
n is 1-3
and non-toxic acid addition salts or hydrates thereof possess antagonistic activity against 5-$HT_3$ receptor, and therefore are useful for the prevention and/or treatment of diseases which are induced when 5-HT acts on 5-$HT_3$ receptors (especially vomiting induced by the administration of anti-cancer agents).

EP 0 405 784 A1

# NOVEL BENZISOQUINOLINE DERIVATIVES

The present invention relates to novel benzisoquinoline derivatives, to processes for their preparation and pharmaceutical compositions containing them.

It is well known that 5-HT (5-hydroxytryptamine, serotonin) is a neurotransmitter in the living body. Three types of receptors which are related to 5-HT are known: $5\text{-}HT_1$, $5\text{-}HT_2$ and $5\text{-}HT_3$ type receptors.

$5\text{-}HT_3$ receptors are widely distributed in the brain, heart and digestive canal, and 5-HT has an activity with respect to these receptors as a mediator.

It is known that when 5-HT acts on $5\text{-}HT_3$ receptors at the peripheral nerve, pain and bradycardia are induced; when 5-HT acts on $5\text{-}HT_3$ receptors at the central nerve, mental activity, e.g. emotions, appetite and memory is induced. 5-HT also acts on $5\text{-}HT_3$ receptors at the CTZ (chemoreceptor trigger zone) in the brain, nausea and vomiting being induced.

It is accordingly believed that $5\text{-}HT_3$ receptor antagonists are useful for the prevention and treatment of central nerve disorders such as schizophrenia, corpulence, mania, and anxiety; gastroenteric functional defects such,as peptic ulcer and peptic oesophagitis; and migraine, vertigo, nausea and vomiting (especially, vomiting induced by administration of anti-cancer agents such as cisplatin.) It is known that large scale administration of Metocraplamide, which acts with side-effects, suppresses vomiting induced by anti-cancer agents. However, other anti-vomiting agents are not effective.

In these circumstances, development of $5\text{-}HT_3$ receptor antagonists began and various companies have developed such compounds, in particular:

(1) Code : ICS-205-930 (Sandoz)
(2) Code : BRL-24924 (Beecham)
(3) Code : GR-38032F (Glaxo)
(4) Code : MDL-72222 (Mellel Dow)

The following specifications disclose compounds related in structure to those of the invention.

. JP 60-214784 (i.e. GB 2153821)
. JP 61-210083 (i.e. EP 191562)
. JP 62-77382 (i.e. EP 219193)
. JP 62-77381 (i.e. EP 210840)
. JP 63-35570 (i.e. GB 2192885)
. JP 63-211279 (i.e. GB 2202530)
. JP 64-22870 (i.e. EP 297651)
. JP 1-311082 (i.e. EP 338650)

For Example, in the specification of Japanese Patent Kokai No. 60-214784, i.e. GB 2153821 the following compounds are disclosed.

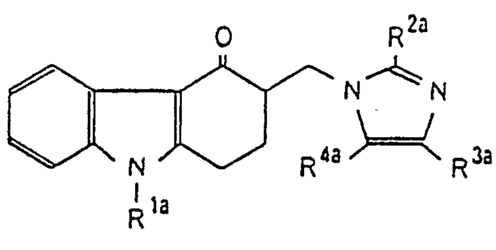

(A)

wherein $R^{1a}$ is hydrogen, C1-10 alkyl, C3-7 cycloalkyl, C3-6 alkenyl, phenyl or phenyl-C1-3alkyl, and one of the groups $R^{2a}$, $R^{3a}$ and $R^{4a}$ is hydrogen, C1-6 alkyl, C3-7 cycloalkyl, C2-6 alkenyl or phenyl-C1-3alkyl and each of the two other groups which may be the same or different, represents hydrogen or C1-6 alkyl.

JP 1-151578 i.e. EP 306323 discloses that the compounds of the formula:

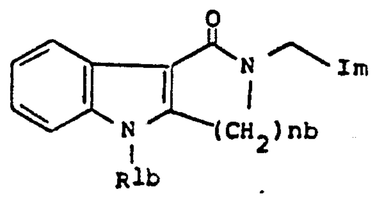

(B)

wherein Im represents an imidazolyl group of the formula:

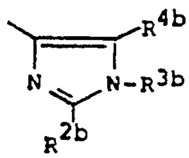

or

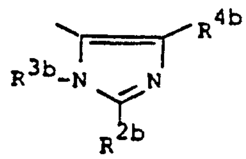

and $R^{1b}$ represents a hydrogen atom or a group selected from $C_{1-6}$alkyl, $C_{3-6}$alkenyl, $C_{3-10}$alkynyl, $C_{3-7}$cycloalkyl, $C_{3-7}$cycloalkyl$C_{1-4}$alkyl, phenyl, phenyl$C_{1-3}$alkyl, phenylmethoxymethyl, phenoxyethyl, phenoxymethyl, $-CO_2R^{5b}$, -COR5$^b$, $-CONR^{5b}R^{6b}$ or $-SO_2R^{5b}$ (wherein $R^{5b}$ and $R^{6b}$, which may be the same or different, each represent a hydrogen atom, a $C_{1-6}$alkyl or $C_{3-7}$cycloalkyl group or a phenyl or phenyl$C_{1-4}$alkyl group, in which the phenyl group is optionally substituted by one or more $C_{1-4}$alkyl, $C_{1-4}$alkoxy or hydroxy groups or halogen atoms, with the proviso that $R^{5b}$ does not represent a hydrogen atom when $R^1$ represents a group $-CO_2R^{5b}$ or $-SO_2R^{5b}$); one of the groups represented by $R^{2b}$, $R^{3b}$ and $R^{4b}$ is a hydrogen atom or a $C_{1-6}$alkyl, $C_{3-7}$cycloalkyl, $C_{3-6}$alkenyl, phenyl or phenyl$C_{1-3}$alkyl group, and each of the other two groups, which may be the same or different, represents a hydrogen atom or a $C_{1-6}$alkyl group;
$n^b$ represents 2 or 3;
and physiologically acceptable salts and solvates thereof, are potent and selective antagonists of the effects of 5-HT at 5-$HT_3$ receptors.

Widespread investigations have been carried out in order to discover compounds which have novel structures and which possess an antagonistic activity on 5-$HT_3$ receptors.

Known 5-$HT_3$ receptor antagonists have a carbazole skeleton, as illustrated in (a) below, or a carboline skeleton, as illustrated in (b) below. The compound GR-38032F (Glaxo), referred to above, has a carbazole skeleton.

(a) 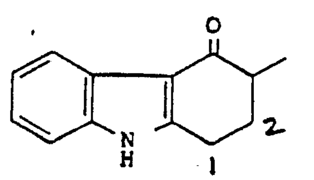 carbazol-4-one

(b) 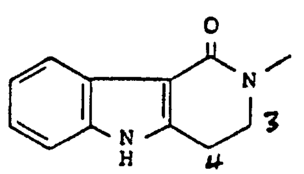 γ-carbolin-1-one

(c) 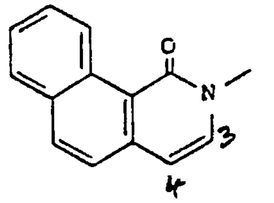 benzisoquinolin-1-one

It will be seen that formula (a) has a 6:5:6 ring member structure with a nitrogen atom in the middle

ring. Formula (b) also has a 6:5:6 structure with one nitrogen atom in the middle ring and a second in the right hand ring.

Both formulae (a) and (b) are characterised by the presence of a single bond in the 1,2-position of the carbazole ring and the (corresponding) 3,4-position of the carboline ring: this feature is consistently present in known 5-$HT_3$ receptor antagonists.

It has now been discovered that new compounds not possessing a carbazole or carboline ring but having instead a benz[1,2-h]isoquinolin-1-one ring of formula (c) illustrated above and, furthermore, having a double bond in the 3,4-position nevertheless possess 5-$HT_3$ receptor antagonistic activity. The benz[1,2-h]-isoquinoline ring having a 6:6:6 ring member structure with one nitrogen atom in the right hand ring is very different chemically to the carbazol-4-one and $\gamma$-carbolin-1-one rings of the prior art compounds.

The presence of a double bond in the 3,4-position further distinguishes the compounds of the present invention.

Among the compounds of the present invention, it has also been found that some compounds possess a stronger antagonistic activity against 5-$HT_3$ receptor than the carbazole type compounds and also possess lower toxicity.

The present invention accordingly provides benzisoquinoline derivatives of the formula:

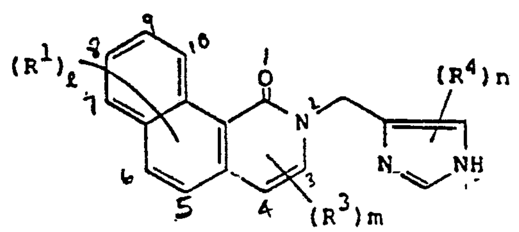

(I)

wherein $R^1$ is hydrogen, C1-4 alkyl, C1-4 alkoxy, hydroxy or halogen;
$R^3$ is hydrogen or C1-4 alkyl;
$R^4$ is hydrogen or C1-6 alkyl;
1 is 1-6;
m is 1 or 2;
n is 1-3;
and non-toxic acid-addition salts and hydrates thereof.

In the formula (I), C1-4 alkyl represented by $R^1$ or $R^3$ means methyl, ethyl, propyl and butyl and isomers thereof.

In the formula (I), C1-6 alkyl represented by $R^4$ means methyl, ethyl, propyl, butyl, pentyl and hexyl and isomers thereof.

In the formula (I), C1-4 alkoxy represented by $R^1$ means methoxy, ethoxy, propoxy and butoxy and isomers thereof.

In the formula (I), halogen represented by $R^1$ means fluorine, chlorine, bromine and iodine.

When l, m or n is greater than 1 the groups $R^1$, $R^3$ and $R^4$ may be the same or different.

In the formula (I), $R^1$, $R^3$ and $R^4$, are preferably hydrogen and methyl.

Throughout the present invention, all of the isomers are included unless otherwise specified. For example, alkyl or alkoxy includes straight or branched chain groups, and the present invention includes isomers generated by the existence of asymmetric carbon atoms, e.g. the existence of certain branched alkyl groups. Also included are tautomers of the compound of formula (I) i.e. where a substituent $R^4$ may be present on either nitrogen atom in the five-membered ring.

The compounds of the formula (I) may be converted into the corresponding acid addition salts. Non-toxic and water-soluble salts are preferable. Suitable salts include the following: salts of inorganic acids e.g. hydrochloride, hydrobromide, sulphate, phosphate and nitrate, and salts of organic acids, e.g. acetate, lactate, tartrate, fumarate, maleate, oxalate, citrate, benzoate, methanesulphonate, ethanesulphonate, benzenesulphonate, toluenesulphonate, isethioate, glucuronate and gluconate. The hydrochloride is preferred.

Compounds of the general formula (I) or salts thereof may be converted into hydrates by conventional means.

According to a feature of the present invention the compounds of formula (I) may be prepared by a process which comprises N-alkylation of a compound of the formula:

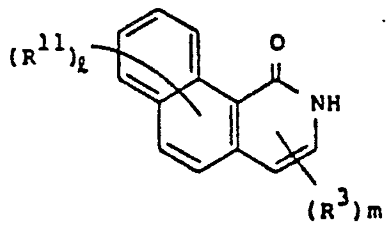

(II)

(wherein $R^{11}$ is hydrogen, C1-4 alkyl, C1-4 alkoxy, protected hydroxy or halogen and the other symbols are as hereinbefore defined) with a compound of the formula:

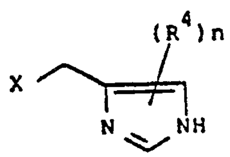

(III)

(wherein X is halogen, and the other symbols are as hereinbefore defined) and when $R^{11}$ is protected hydroxy, subjecting the compound thus obtained by hydrolysis under acidic conditions to convert the group $OR''$ to hydroxy.

N-alkylation is a known reaction and may be carried out, for example, in a polar organic solvent (e.g.diethyl ether, THF, acetonitrile, DMF and HMPA), in the presence of a base (e.g. sodium hydride).

Suitable hydroxy-protecting groups are those which can be removed under acidic conditions, for example, methoxymethyl, tetrahydrofuranyl, tetrahydropyranyl or 1-methoxyethyl.

Hydrolysis under acidic condition is a known reaction and may be carried out, for example, in a water-miscible organic solvent (e.g. methanol, ethanol, THF or dioxan), using an aqueous solution of organic acid (e.g. acetic acid, p-toluenesulphonic acid, trichloroacetic acid or oxalic acid) or an aqueous solution of inorganic acid (e.g. hydrochloric acid, sulphuric acid or hydrobromic acid) or mixture thereof, at a temperature of from 0 °C - 90 °C.

The compound of formula (II) may be prepared as shown in the following reaction Scheme (A).

Each symbol has the following meaning or is as hereinbefore defined.

$R^{30}$, $R^{31}$ ; hydrogen or C1-4 alkyl

$R^{50}$ ; C1-4 alkyl

Scheme (A)

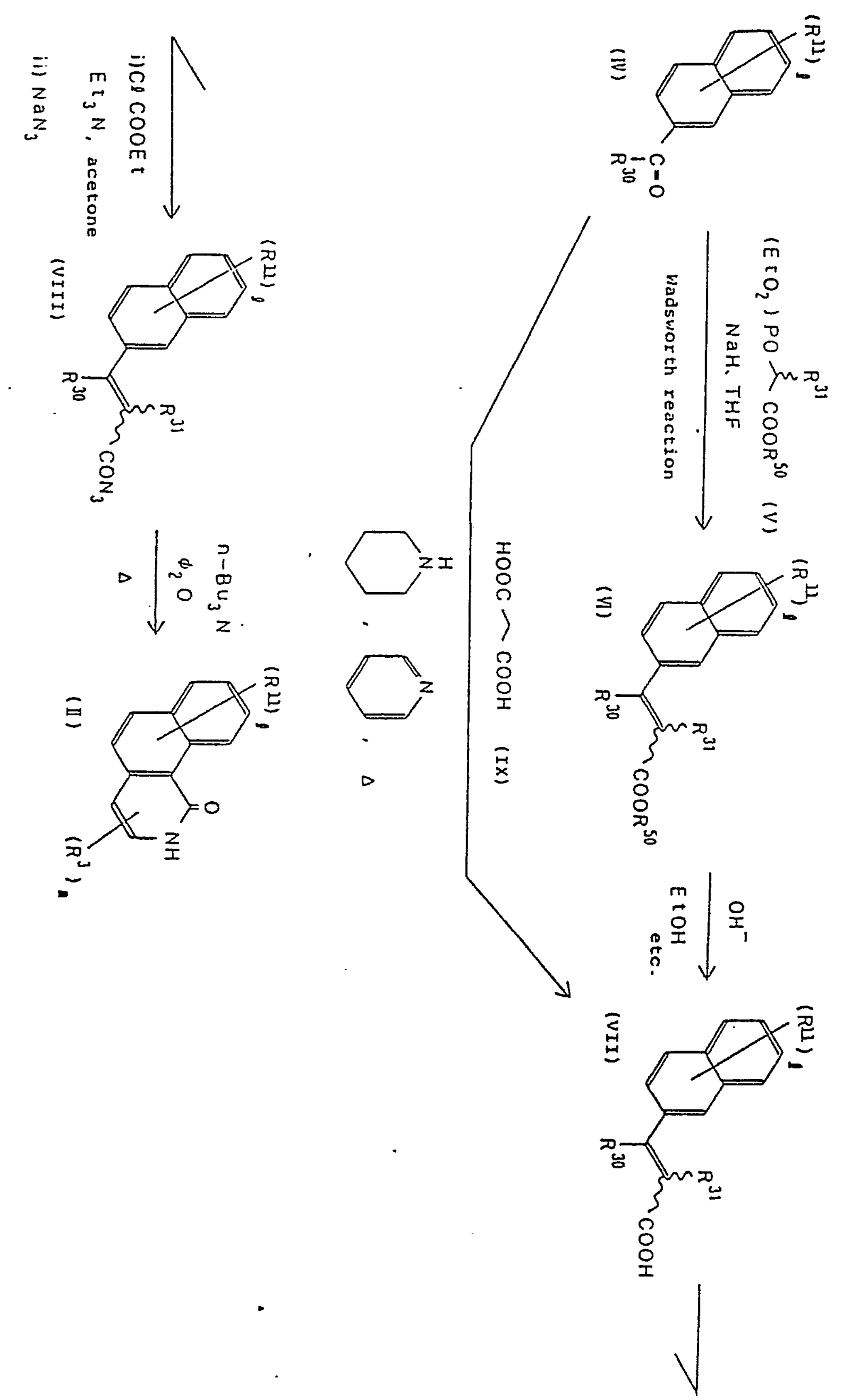


(IV) (V) (VI) (VII) (VIII) (IX) (II)

Starting materials and reagents used in the process of the present invention are known per se or may be prepared by known methods.

For example, the compound of formula (III) wherein $(R^4)_n$ is 5-methyl and X is chlorine is commercially available. The compound of formula (IV) wherein $R^{30}$ is hydrogen and $(R^{11})1$ is hydrogen is described in Chem. Abst., 59 , 3899b (1963).

The compounds of the present invention of formula (I) possess antagonistic activity against 5-$HT_3$ receptors as described above. In the standard laboratory test described below the results in the following table I were obtained.

Table I

| Antagonistic activity against 5-$HT_3$ receptors in vivo in rats | | |
|---|---|---|
| Example No. of the compound | $IC_{50}$ i.v. (μg/kg) | $IC_{50}$ i.d. (μg/kg) |
| 1 | 0.29 | 7.5 |
| 1(a) | 2.4 | -- |
| 1(d) | 0.89 | -- |
| 1(e) | 1.19 | -- |
| 1(f) | 0.62 | -- |
| 1(g) | 5.87 | -- |

Experimental method

A male Wistar rat was urethane-anaesthetized and fixed. Cannulas were inserted in the carotid artery and in the thigh vein for use in the recording of blood pressure and heart beat, and for the administration of the compounds, respectively.

Various amounts of a compound of the present invention were administered to the vein or to the duodenum. 5-HT was administered rapidly to the vein, 2 mins after insertion of the cannulas. The suppressive effect of the compounds was confirmed by the measurement of the reflex bradycardia generated. (Nature 316, 11 (1985))

Tests confirmed that the toxicity of the compounds of the present invention is very low. Therefore, the compounds of the present invention may be considered to be sufficiently safe and suitable for pharmaceutical use.

For example, the value of acute toxicity ($LD_{50}$) of the compound prepared in example 1 was 94 mg/kg animal body weight by intravenous administration and more than 800 mg/kg animal body weight by oral administration in mice.

The compounds of the present invention of formula (I) possess antagonistic activity against 5-$HT_3$ receptors, shown in vivo by the experimental results above, and are expected to be useful for the uses arising from that activity.

It is believed that to block the activity of 5-$HT_3$ receptors is useful for the prevention and the treatment of central nerve diseases such as schizophrenia, corpulence, mania and anxiety; gastroenteric functional defects such as peptic ulcer and peptic oesophagitis; and migraine, vertigo, nausea and vomiting (especially, vomiting induced by administration of anti-cancer agents such as cisplatin) in animals, especially in human beings.

For the purposes described above, the compounds of the present invention of formula (I), a non-toxic addition salt thereof or a hydrate thereof, will normally be administered systemically or partially, generally by oral or parenteral administration.

The present invention also provides a pharmaceutical composition which comprises a benzisoquinoline derivative of general formula (I) or a non-toxic acid addition salt or hydrate thereof in association with a pharmaceutically acceptable carrier or coating.

The present invention also provides a method for the prevention or treatment of a mammalian host (e.g.

a human or animal patient) suffering from or subject to a disorder arising from the action of 5-hydroxytryptamine(5-HT) or 5-$HT_3$ receptors which method comprises the administration of an effective amount of a benzisoquinoline derivative of general formula (I) or a non-toxic acid addition salt or hydrate thereof.

The doses to be administered are dependant upon for example age, body weight, symptoms, the desired therapeutic effect, the route of administration, and the duration of the treatment. In the human adult, the dose per person is generally from 50 µg to 100 mg, preferably from 1 mg to 20 mg, by oral administration, up to several times per day, and from 5 µg to 10 mg, preferably 500 µg to 10 mg, by parenteral administration up to several times per day, or continuous administration for periods of time from 1 to 24 hrs per day intravenously.

As mentioned above, the doses used are dependant upon various factors. Therefore, there are cases in which a dose lower than or greater than the ranges specified above may be used.

Administration of the compounds of the present invention may be in the form of solid compositions, liquid compositions or other compositions for oral administration and as for e.g. injections, liniments or suppositories for parenteral administration.

Solid compositions for oral administration include tablets, pills, capsules, dispersible powders and granules.

Capsules include soft capsules and hard capsules.

Liquid compositions for oral administration include pharmaceutically acceptable-emulsions, solutions, suspensions, syrups and elixirs. Moreover such compositions may be employed with inert diluent(s) such as water and ethanol.

Injections for parenteral administration include sterile aqueous or non-aqueous solutions, suspensions and emulsions.

Other compositions for parenteral administration include liquids for external use, e.g. external solutions, endermic liniments, ointments, suppositories for rectal administration and pessaries.

The following Examples and Reference Examples serve to illustrate the present invention.

The solvents in parentheses show the developing or eluting solvents and the ratio of the solvents used by volume in chromatographic separations.

Unless otherwise specified, "IR" were measured by the KBr disk method.

The compounds of the present invention are named as benz [1,2-h] isoquinoline derivatives with numbering as shown below;

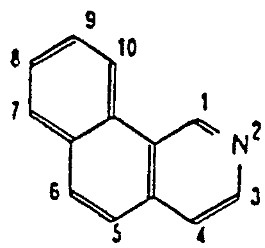

Reference Example 1

Synthesis of 3-(2-naphthyl)acrylic acid

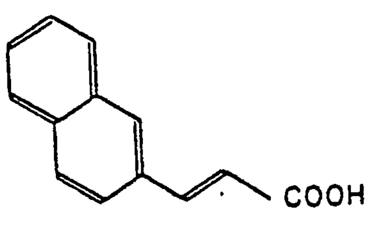

A solution of 2-naphthylaldehyde (3 g), piperidine (0.49 ml) and malonic acid (4.0 g) in pyridine (15 ml)

was stirred for 2 hrs at 120°C. After cooling, the solution was acidified with conc. sulphuric acid. The solution was extracted with EtOAc. The oily layer was washed, dried, and evaporated to give the title compound (3.6 g) having the following physical data:
TLC : Rf 0.15 (EtOAc : hexane = 1 : 1).

Reference Example 2

Synthesis of 3-(2-naphthyl)acryloyl azide

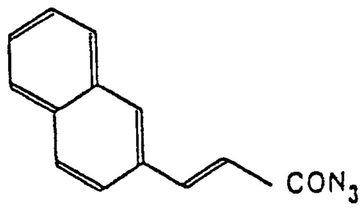

A solution of the compound prepared in reference example 1 (991 mg) and $Et_3N$ (0.84 ml) in acetone (5 ml) was cooled with ice. Ethyl chloroformate (0.57 ml) was added dropwise to the solution. The solution of sodium azide (488 mg) in water (2 ml) was added dropwise to the solution. Acetone (5 ml) was added to the mixture. The solution was stirred for 1 hr. After reaction, solvent was removed from the solution. The residue was poured into water. The mixture was extracted with methylene chloride. The oily layer was washed, dried and evaporated to give the title compound.

Reference Example 3

Synthesis of 1,2-dihydrobenz[1,2-h]isoquinolin-1-one

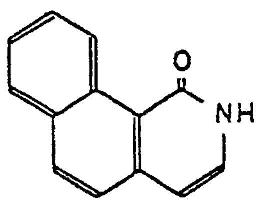

A solution of n-$BU_3N$ (1.3 ml) in diphenyl ether (5 ml) was heated at 230°C. A solution of the compound prepared in reference example 2 in diphenyl ether was added to the solution dropwise. After cooling, hexane was added to the reaction solution. The solids deposited were gathered by filtration. Solids were washed with hexane, dried to give the title compound (655 mg) having the following physical data.
TLC : Rf 0.58 (chloroform : methanol = 5 : 1).

Example 1

Synthesis of 2-(5-methylimidazol-4-ylmethyl)-1,2-dihydrobenz[1,2-h]isoquinolin-1-one hydrochloride

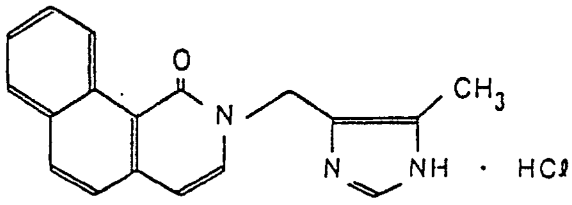

The compound prepared in reference example 3 (293 mg) was dissolved in DMF (4 ml). Sodium hydride (240 mg) was added to the solution. The mixture was stirred for 20 mins at room temperature. A suspension of 4-chloromethyl-5-methylimidazole hydrochloride (501 mg) in DMF (2 ml) was added to the solution. The mixture was stirred for 40 mins at room temperature. The reaction mixture was poured into ice-water. The mixture was extracted with chloroform. The oily layer was dried, and evaporated. The residue was purified by column chromatography on silica gel (chloroform : methanol = 20 : 1) to give pale yellow oil (204 mg). The oil was dissolved in methanol (2 ml), 4N HCl-dioxane (2 ml) was added to the solution. The solution was evaporated. The residue was washed with diethyl ether, dried to give the title compound (194 mg) having the following physical data.

TLC : Rf 0.38 (chloroform : methanol = 5 : 1);

IR : $\nu$ 3087, 2992, 2823, 2755, 2652, 1653, 1611, 1547, 1478, 1425, 1382, 1243, 1137, 835, 745, 631, 517 $cm^{-1}$.

Example 1(a) - 1(g)

By the same method shown in Reference Examples 1 to 3 and by the same procedure of Example 1, the compounds having the physical data described in following Table II were obtained.

The compound shown in Example 1(f) was obtained by treating with conc. HCl-MeOH before conversion into the acid addition salt.

Table II :

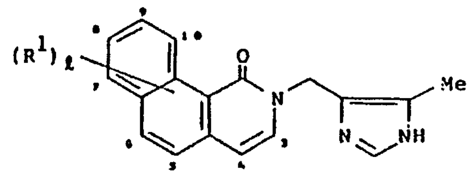

| Example No. | $(R^1)_\ell$ | Name | TLC (Rf) | IR ($\nu$ $cm^{-1}$) |
|---|---|---|---|---|
| 1(a) | 5-MeO- | 2-(5-methylimidazol-4-ylmethyl)-5-methoxy-1,2-dihydrobenz[1,2-h]isoquinolin-1-one hydrochloride | 0.52 ($CHCl_3$ : MeOH = 5 : 1) | 3008, 1654, 1613, 1553, 1503, 1463, 1380, 1292, 1243, 1175, 1115, 1052, 846, 811, 739 |
| 1(b) | 5-MeO-<br>7-MeO- | 2-(5-methylimidazol-4-ylmethyl)-5,7-dimethoxy-1,2-dihydrobenz[1,2-h]isoquinolin-1-one hydrochloride | 0.25 ( $CHCl_3$ : MeOH = 5 : 1 ) | 3396, 3004, 2904, 1656, 1603, 1505, 1468, 1276, 1254, 1242, 1105, 1060, 818, 761, 734 |
| 1(c) | 8-MeO- | 2-(5-methylimidazol-4-ylmethyl)-8-methoxy-1,2-dihydrobenz[1,2-h]isoquinolin-1-one hydrochloride | 0.57 ( $CHCl_3$ : MeOH = 5 : 1 ) | 2991, 2750, 1654, 1605, 1552, 1488, 1380, 1362, 1252, 1169, 1133, 1045, 850, 836 |

Table II : (continue)

| Example No. | $(R^1)_\ell$ | Name | TLC (Rf) | IR ($\nu cm^{-1}$) |
|---|---|---|---|---|
| 1(d) | 6-Me- | 2-(5-methylimidazol-4-ylmethyl)-6-methyl-1,2-dihydrobenz[1,2-h]isoquinolin-1-one hydrochloride | 0.3 ($CHCl_3$ : MeOH = 10 : 1) | 2993, 2839, 2755, 2653, 1655, 1610, 1549, 1383, 1241, 882, 836, 759 |
| 1(e) | 5-Me- | 2-(5-methylimidazol-4-ylmethyl)-5-methyl-1,2-dihydrobenz[1,2-h]isoqionolin-1-one hydrochloride | 0.33 ( $CHCl_3$ : MeOH = 10 : 1 ) | 2980, 2810, 2739, 2647, 1655, 1602, 1443, 1375, 1241, 1213, 1175, 1023, 921, 865, 811, 755 |
| 1(f) | 5-HO- | 2-(5-methylimidazol-4-ylmethyl)-5-hydroxy-1,2-dihydrobenz[1,2-h]isoquinolin-1-one hydrochloride | 0.30 ( $CHCl_3$ : MeOH = 5 : 1 ) | 3156, 1650, 1616, 1581, 1448, 1337, 1240, 1177, 1154, 982, 846, 813, 744 |

Table II : (continue)

| Example No. | $(R^1)_\ell$ | N a m e | T L C (Rf) | I R ( ν $cm^{-1}$) |
|---|---|---|---|---|
| 1(g) | 6-Br- | 2-(5-methylimidazol-4-ylmethyl)-6-bromo-1,2-dihydrobenz[1,2-h]isoquinolin-1-one hydrochloride | 0.45 ( $CHCl_3$ : MeOH = 10 : 1 ) | 2992, 2838, 2753, 2654, 1665, 1609, 1586, 1545, 1479, 1402, 1240, 905 |

Formulation Example 1

The following components were admixed in conventional method and punched out to obtain 100 tablets each containing 20 mg of active ingredient.

| | |
|---|---|
| * 2-(5-methylimidazol-4-ylmethyl)-1,2-dihydrobenz[1,2-h]isoquinolin-1-one hydrochloride --- | 1.0 g |
| * Calcium cellulose glycolate (carboxymethylcellulose calcium; disintegrating agent) --- | 0.2 g |
| * Magnesium stearate (lubricating agent) - | 0.1 g |
| * Lactose --- | 8.7 g |

Formulation Example 2

The following components were admixed in conventional manner. The solution was sterilized in conventional manner, and 1 ml portions were placed into 5 ml ampoules and freeze-dried to obtain 100 ampoules each containing 2 mg of the active ingredient.

| | |
|---|---|
| * 2-(5-methylimidazol-4-ylmethyl)-1,2-1,2-dihydrobenz[1,2-h]isoquinolin-1-one hydrochloride --- | 0.2 g |
| * lactose --- | 2 g |
| * distilled water --- | 100 ml |

**Claims**

1. A benzisoquinoline derivative of the formula:

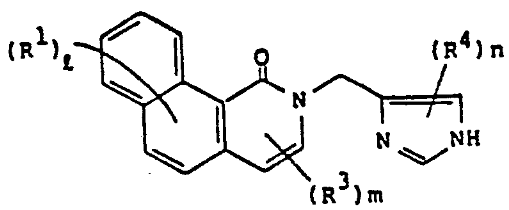

(I)

wherein $R^1$ is hydrogen, C1-4 alkyl, C1-4 alkoxy, hydroxy or halogen:
$R^3$ is hydrogen or C1-4 alkyl;
$R^4$ is hydrogen or C1-6 alkyl;
l is 1-6;
m is 1 or 2;
n is 1-3
or a non-toxic acid addition salt or hydrate thereof.

2. A compound according to claim 1 wherein $R^1$, $R^3$ and $R^4$, which may be the same or different, represent hydrogen or methyl.

3. A compound according to claim 1 which is 2-(5-methylimidazol-4-ylmethyl)-1,2-dihydrobenz[1,2-h]-isoquinolin-1-one, 2-(5-methylimidazol-4-ylmethyl)-5-methoxy-1,2-dihydrobenz[1,2-h]isoquinolin-1-one, 2-(5-methylimidazol-4-ylmethyl)-5,7-dimethoxy-1,2-dihydrobenz[1,2-h]isoquinolin-1-one, 2-(5-methylimidazol-4-ylmethyl)-8-methoxy-1,2-dihydrobenz[1,2-h]isoquinolin-1-one, 2-(5- methylimidazol-4-ylmethyl)-6-methyl-1,2-dihydrobenz[1,2-h]isoquinolin-1-one, 2-(5-methylimidazol-4-ylmethyl)-5-methyl-1,2-dihydrobenz[1,2-h]-isoquinolin-1-one, 2-(5-methylimidazol-4-ylmethyl)-5-hydroxy-1,2-dihydrobenz[1,2-h]isoquinolin-1-one and 2-(5-methylimidazol-4-ylmethyl)-6-bromo-1,2-dihydrobenz[1,2-h]isoquinolin-1-one, or a non-toxic acid addition salt thereof.

4. A process for the preparation of a compound of formula:

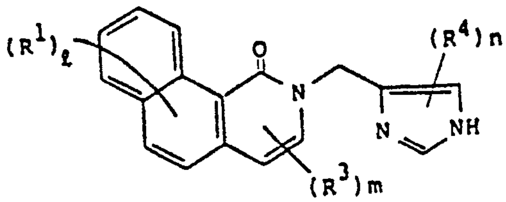

(I)

(wherein the various symbols are as defined in claim 1) which comprises N-alkylation of a compound of the formula:

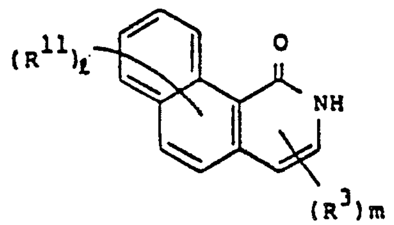

(II)

(wherein $R^{11}$ is hydrogen, C1-4 alkyl, C1-4 alkoxy, halogen or a protected hydroxy group; and the other symbols are as defined in claim 1 with a compound of the formula:

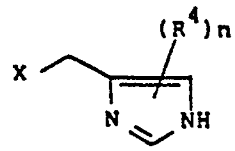

(III)

(wherein X is halogen; and the other symbols are as defined in claim 1 and, if necessary, subjecting the resulting compound to hydrolysis under acidic condition, to convert a protected hydroxy group to hydroxy.

5. A pharmaceutical composition which comprises a benzisoquinoline derivative of the formula (I), a non-toxic acid addition salt or hydrate thereof in association with a pharmaceutically acceptable carrier or coating.

6. A benzisoquinoline derivative according to claim 1 or a pharmaceutically acceptable salt or hydrate thereof for use as a medicament.

Claims for the following Contracting States: ES, GR

1. A process for the preparation of a benzisoquinoline derivative of the formula:

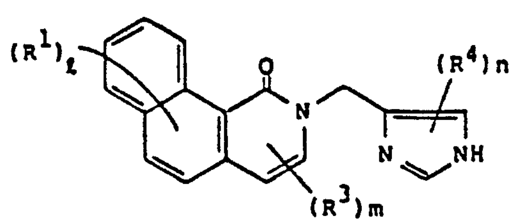

(I)

wherein $R^1$ is hydrogen, C1-4 alkyl, C1-4 alkoxy, hydroxy or halogen:
$R^3$ is hydrogen or C1-4 alkyl;
$R^4$ is hydrogen or C1-6 alkyl;

l is 1-6;
m is 1 or 2;
n is 1-3
or a non-toxic acid addition salt or hydrate thereof, which process comprises N-alkylation of a compound of the formula:

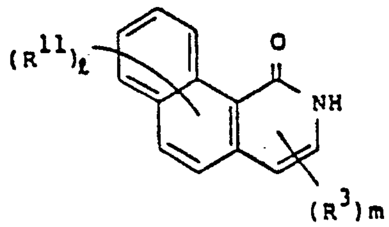

(II)

(wherein $R^{11}$ is hydrogen, C1-4 alkyl, C1-4 alkoxy, halogen or a protected hydroxy group) and the other symbols are as hereinbefore defined with a compound of the formula:

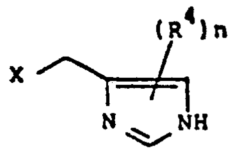

(III)

(wherein X is halogen; and the other symbols are as hereinbefore defined and, if necessary, subjecting the resulting compound to hydrolysis under acidic condition, to convert a protected hydroxy group to hydroxy.
2. A process according to claim 1 wherein $R^1$, $R^3$ and $R^4$, which may be the same or different, represent hydrogen or methyl.
3. A process according to claim 1 in which the benzisoquinoline derivative of formula (I) is 2-(5-methylimidazol-4-ylmethyl)-1,2-dihydrobenz[1,2-h]isoquinolin-1-one, 2-(5-methylimidazol-4-ylmethyl)-5-methoxy-1,2-dihydrobenz[1,2-h]isoquinolin-1-one, 2-(5-methylimidazol-4-ylmethyl)-5,7-dimethoxy-1,2-dihydrobenz[1,2-h]isoquinolin-1-one, 2-(5-methylimidazol-4-ylmethyl)-8-methoxy-1,2-dihydrobenz[1,2-h]-isoquinolin-1-one, 2-(5-methylimidazol-4-ylmethyl)-6-methyl-1,2-dihydrobenz[1,2-h]isoquinolin-1-one, 2-(5-methylimidazol-4-ylmethyl)-5-methyl-1,2-dihydrobenz[1,2-h]isoquinolin-1-one, 2-(5-methylimidazol-4-ylmethyl)-5-hydroxy-1,2-dihydrobenz[1,2-h]isoquinolin-1-one and 2-(5-methylimidazol4-ylmethyl)-6-bromo-1,2-dihydrobenz[1,2-h]isoquinolin-1-one, or a non-toxic acid addition salt thereof.
4. A process for the preparation of a pharmaceutical composition which comprises formulating a benzisoquinoline derivative of the formula (I), a non-toxic acid addition salt or hydrate thereof with a pharmaceutically acceptable carrier or coating.
5. A benzisoquinoline derivative according to claim 1 or a pharmaceutically acceptable salt or hydrate thereof for use as a medicament.

European Patent Office

# EUROPEAN SEARCH REPORT

Application number

EP 90306389.9

**DOCUMENTS CONSIDERED TO BE RELEVANT**

| Category | Citation of document with indication, where appropriate, of relevant passages | Relevant to claim | CLASSIFICATION OF THE APPLICATION (Int Cl 5) |
|---|---|---|---|
| A | EP - A2 - 0 247 760 (SCHERING) * Formula I * | 1 | C 07 D 401/06<br>A 61 K 31/47 |
| A | CHEMICAL ABSTRACTS, vol. 95, no. 8, August 24, 1981 Columbus, Ohio, USA WOLFBEIS, OTTO S. et al. "Syntheses of fluorescent dyes. 10. 2-Substituted pyrano(2,3-c)isoquinoline-3,6-diones and merocyanine dyes from homophthalimides" page 71, column 1, abstract-no. 63 658c & (Inst. Org. Chem., Univ. Graz, A-8010 Graz, Austria). Liebigs Ann. Chem. 1981, (5), 811-18 (Ger). | 1 | |
| A | CHEMICAL ABSTRACTS, vol. 108, no. 3, January 18, 1988 Columbus, Ohio, USA CARBARES, JACQUES et al. "Utilization of dimethyl 4-aryl(aralkyl)-1-acetonyl-7-oxabicyclo(2.2.1)hept-2-ene-2,3-dicarboxylates. Access to new heterocyclic compounds" page 587, column 2, abstract -no. 21 750h & (Lab. Chim. Org., CEPM, 49045 Angers, Fr.). Bull. Soc. Chim. Fr. 1987, (2), 339-49 (Fr). | 1 | TECHNICAL FIELDS SEARCHED (Int Cl 5)<br>C 07 D 401/00 |
| A | CHEMICAL ABSTRACTS, vol. 86, no. 7, February 14, 1977 Columbus, Ohio, USA | | |

The present search report has been drawn up for all claims

| Place of search | Date of completion of the search | Examiner |
|---|---|---|
| VIENNA | 30-08-1990 | HAMMER |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82

European Patent Office

# EUROPEAN SEARCH REPORT

Application number

-2-
EP 90306389.9

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int Cl⁵) |
| | NONOMIYA, ICHIYA et al. "Photocyclization of enamides. Part XI. Syntheses of benzo(i)phenanthridines related to aza steroids" page 519, column 1, abstract -no. 43 531s & (Kobe Women's Coll. Pharm., Kobe, Japan). J. Chem. Soc., Perkin Trans. 1 1976, (17), 1868-72 (Eng). Photochem. ---- | | |
| | | | TECHNICAL FIELDS SEARCHED (Int Cl⁵) |

The present search report has been drawn up for all claims

| Place of search | Date of completion of the search | Examiner |
|---|---|---|
| VIENNA | 30-08-1990 | HAMMER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82